# FASCICULE DE BREVET EUROPEEN

(11) **EP 2 953 922 B1**
(45) Date de publication et mention de la délivrance du brevet: **23.08.2017**
(21) Numéro de dépôt: 14706907.4
(22) Date de dépôt: 10.02.2014
(51) Int. Cl.: C07C 67/333, C07C 253/30, C07C 51/353

(54) **SYNTHESE DE COMPOSE INSATURE RAMIFIE PAR METATHESE CROISEE**
SYNTHESE EINER VERZWEIGTEN UNGESÄTTIGTEN VERBINDUNG MITTELS KREUZMETATHESE
SYNTHESIS OF A BRANCHED UNSATURATED COMPOUND BY MEANS OF CROSS METATHESIS

(30) Priorité: 08.02.2013 FR 1351101
(43) Date de publication de la demande: 16.12.2015
(73) Titulaire: Arkema France, 92700 Colombes (FR)
(72) Inventeur: DUBOIS, Jean-Luc, 69390 Millery (FR); COUTURIER, Jean-Luc, 69006 Lyon (FR)
(74) Mandataire: Brevalex
(86) Numéro de dépôt international: PCT/FR2014/050248
(87) Numéro de publication internationale: WO 2014/122411

(56) Documents cités:
- NSTOR M CARBALLEIRA ET AL: "First total synthesis and antileishmanial activity of ()-16-methyl-11-heptadecenoic acid, a new marine fatty acid from the sponge", CHEMISTRY AND PHYSICS OF LIPIDS, LIMERICK, IR, vol. 164, no. 2, 24 novembre 2010 (2010-11-24), pages 113-117, XP028358858, ISSN: 0009-3084, DOI: 10.1016/J.CHEMPHYSLIP.2010.11.006 [extrait le 2010-12-01]
- HANS JÜRGEN BESTMANN ET AL: "Pheromone, VII. Synthese von 1-substituierten (Z)-9-Alkenen", CHEMISCHE BERICHTE, vol. 108, no. 11, 1 novembre 1975 (1975-11-01), pages 3582-3595, XP055073059, ISSN: 0009-2940, DOI: 10.1002/cber.19751081120
- URSULA BIERMANN ET AL: "Synthesis of alkyl-branched fatty acids", EUROPEAN JOURNAL OF LIPID SCIENCE AND TECHNOLOGY, vol. 110, no. 9, 1 septembre 2008 (2008-09-01), pages 805-811, XP055084228, ISSN: 1438-7697, DOI: 10.1002/ejlt.200800033 cité dans la demande

## Description

### DOMAINE DE L'INVENTION

L'invention a pour objet un procédé de synthèse de composé acide, ester ou nitrile gras, insaturé et ramifié mettant en oeuvre au moins une réaction de métathèse.

Au sens de l'invention, on entend par composé :
- « acide, ester ou nitrile » : un composé aliphatique porteur d'au moins une fonction terminale acide, ester ou nitrile ;
- « gras » : un composé aliphatique dont la chaine principale la plus longue comporte de 8 à 36 atomes de carbone , de préférence de 8 à 26, de préférence de 10 à 24 atomes de carbone ;
- « insaturé » : un composé aliphatique qui comporte une ou plusieurs doubles liaisons carbone-carbone ;
- « ramifié » ou « branché » : un composé aliphatique à chaîne principale linéaire comportant au moins une autre chaîne carbonée (ou « ramification ») de nombre de carbone(s) compris dans la gamme de 1 à 11, de préférence de 1 à 10, de préférence de 1 à 5, sur au moins un des carbones de la chaîne principale ;

Et on utilise l'abréviation « composé gras insaturé ramifié » pour désigner tout « composé acide, ester ou nitrile gras insaturé ramifié » selon l'invention.

### ARRIERE PLAN TECHNIQUE

Les acides gras ramifiés existent dans la nature (http://www.cyberlipid.org/cyberlip/home0001.htm), mais sont extrêmement rares, peu disponibles commercialement et particulièrement coûteux. Pour illustrer la rareté de ces produits, on peut relever que le prix des produits de cette catégorie est bien supérieur à celui des produits non ramifiés, dans le catalogue de la Société Aldrich par exemple http://www.sigmaaldrich.com/catalog/product/sigma/m6281?lang=fr&region=FR ..

Les procédés de synthèse de ces acides gras insaturés et ramifiés décrits dans la littérature sont divers. On peut citer l'isomérisation des acides gras en catalyse hétérogène ; ce procédé conduit toutefois à une large distribution de divers acides gras branchés. On peut citer à ce sujet l'article de U. Biermann et al. dans Eur. J. Lipid Sci. Technology, intitulé « Synthesis of alkyl branched fatty acids » 2008,110,805-811 et celui de Zhang et al., paru dans The proceedings of the 3rd International Conference on Functional Molecules, intitulé « Novel Process to Produce Branched Fatty Acid/Ester for Biodiesel and New Surfactant».

Diverses autres méthodes telles que l'alkylation des oléfines, les procédés d'hydroformylation et hydrogénation, les procédés de dimérisation des acides gras, les réactions de Friedel-Kraft, les réactions impliquant du formaldéhyde (ENE-reaction), les additions radicalaires, ont été étudiées comme en témoigne l'article d'Ivan Malchev, intitulé «Plant Oil based Lubricants » disponible auprès du Department of Plant Agriculture, Ontario Agriculture College, University of Guelph 50 Stone Road W., Guelph, Ontario, Canada N1G 2W1 et téléchargé sur Internet *de* *http:*//*ebookbrowse.com*/*i-malchev-pdf-d92064738 et* *http:*//*www.pdfio.com*/*k-1103149.html.* Cependant, il faut noter que globalement aucune de ces méthodes ne donne complètement satisfaction. Les produits obtenus par isomérisation catalytique sont des mélanges d'isomères ; les produits obtenus par hydroformylation sont des saturés et on perd ainsi certaines propriétés à froid ; les produits d'addition, notamment radicalaire, sont des mélanges, et il n'est pas possible obtenir un produit bien spécifique.

Une autre démarche récente, notamment décrite dans le document de brevet EP1019512 consiste à identifier les gènes dans les plantes qui conduisent à des acides ramifiés et à les transférer à des microorganismes pour leur faire produire des acides spécifiques. Mais cette méthode n'en est qu'à ses balbutiements.

On peut encore citer les synthèses décrites dans l'article de N.M. Carballeira et al., publié dans Chemistry and Physics of Lipids, 2011, 164, 113-117, intitulé "First total synthesis and antileishmanial activity of (Z)-16-methyl-11-heptadecenoic acid, a new marine fatty acid from the sponge Dragmaxia undata" ou celui de H.J. Bestmann, Chem. Ber, 1975, 108, 3582-3595, intitulé "Synthesis of 1-substitued (Z)-9-alkenes"*.*

Les composés ramifiés (ou « branchés ») se distinguent des composés linéaires par des points de fusion plus bas et cette propriété offre certains débouchés sur le plan industriel. On peut citer à ce sujet l'acide isostéarique, produit commercial, obtenu comme sous-produit du procédé de dimérisation de l'acide oléique par catalyse avec la montmorillonite.

L'influence de l'acide isostéarique sur les propriétés d'étalement, de viscosité et de stabilité à l'oxydation et à l'hydrolyse des produits trouve des applications dans les domaines de la cosmétique et de la lubrification (voir l'article de Biermann et al. cité précédemment).

En général, la stratégie retenue par les industriels du secteur de l'oléochimie privés d'un accès aux composés branchés en concentration suffisante, est d'utiliser comme alternative des composés insaturés qui offrent, eux aussi, des points de fusion plus bas que ceux des composés linéaires saturés correspondants. Cependant, les composés insaturés sont aussi moins stables, et sensibles à l'oxydation.

Il existe donc un réel besoin de mettre au point un procédé de synthèse simple et rapide (comportant le moins d'étapes possible) de composés gras ramifiés, notamment des acides gras insaturés ramifiés.

De manière connue, la métathèse croisée consiste à faire réagir, en présence d'un catalyseur de métathèse, deux molécules insaturées selon le processus réactionnel schématique suivant :
A₁A₂ - C=C - B₁B₂ + D₁D₂ - C=C - E₁E₂ ⇔
A₁A₂ - C=C - D₁D₂ + A₁A₂ - C=C - E₁E₂ + B₁B₂- C=C - D₁D₂ + B₁B₂ - C=C - E₁E₂ (métathèse croisée) et,
A₁A₂ - C=C - A₁A₂ + B₁B₂ - C=C - B₁B₂ + D₁D₂ - C=C - D₁D₂ + E₁E₂ - C=C - E₁E₂ (homométathèse)

La métathèse croisée est utilisée avec l'objectif de synthétiser un produit cible, par exemple A₁A₂ - C=C - D₁D₂ dans l'équation ci-dessus, mais qui sera accompagné le cas échéant des trois autres composés possibles issus de la métathèse croisée des 2 mêmes réactifs, ainsi que de certains composés issus de l'homo-métathèse de chaque réactif, réaction qui accompagne la réaction principale.

L'utilisation de la métathèse a été décrite par la société ELEVANCE pour la fabrication de bases de carburants, notamment carburants d'avion, à partir d'huiles végétales. Ainsi, les documents de brevet US2011/0113679 et US2011/0237850 décrivent la réaction entre une huile végétale naturelle et un mélange d'oléfines légères, conduisant, après traitement complémentaire, à un mélange de composés dont le nombre d'atomes de carbone est variable, multiple et non contrôlé, et dont les propriétés en termes de densité énergétique et de point éclair conviennent pour la fabrication de carburants. Néanmoins, ce type de mélange ne convient pas pour des applications plus exigeantes en termes notamment de pureté. De plus, le procédé décrit dans chacun de ces documents ne permet pas de fabriquer économiquement des composés de bas point de fusion, c'est-à-dire de température de fusion (Tf) inférieure à 0°C, de préférence inférieure à -20 °C, de manière encore plus préférée inférieure à -30 °C.

La présente invention vise au contraire à fournir un procédé dans lequel la sélectivité, la pureté, et donc les propriétés physicochimiques du composé gras insaturé ramifié sont contrôlées, pour pouvoir l'utiliser dans des applications de haute performance technique, telles que les fluides diélectriques, les tensioactiactifs de spécialité, les émulsifiants, les agents de friction, les additifs antistatiques, additifs anti-buée, les agents de démoulage, les dispersants de pigments, les lubrifiants haute performance, les cires et émulsifiants de cires, les additifs de transformation de polymères, les stabilisants de PVC, les encres, résines, peintures, vernis, les solvants, les rouges à lèvres, les crèmes pour la peau, les déodorants, notamment sous forme de sticks, les colorants pour cheveux, les shampoings et autres savons liquides, les mousses à raser, les lessives, les nettoyants, les adoucissants textiles, etc.

La Demanderesse a maintenant trouvé un moyen de rendre facilement disponibles des composés insaturés ramifiés, par un procédé utilisant, dans certaines conditions, une réaction de métathèse croisée entre au moins deux réactifs sélectionnés selon l'invention et permettant de cibler plus spécialement la synthèse de composés acides, esters ou nitriles gras insaturés ramifiés. De façon inattendue, le procédé selon l'invention favorise la synthèse du produit cible, c'est-à-dire la synthèse de composé gras insaturé ramifié avec un rendement d'au moins 70%, compatible avec des applications industrielles.

### RESUME DE L'INVENTION

La présente invention a donc pour objet un procédé de synthèse de composé acide, ester ou nitrile gras insaturé ramifié parfaitement ciblé par la mise en oeuvre de réactifs sélectionnés dans une réaction de métathèse croisée.

En particulier, l'invention a pour objet un procédé de synthèse de **composé gras insaturé ramifié, de formule III** : A-CH=CB'-B dans laquelle,
A est un radical constitué d'une chaîne alkyle linéaire, éventuellement insaturée, comprenant de 1 à 20 atomes de carbone et comportant une fonction terminale acide, ester (de mono-alcool ou de polyol), ou nitrile,
B' est H ou une chaine alkyle comportant de 1 à 10 atomes de carbone, et
B est :
- si B' est une chaine alkyle, B est une chaine alkyle comportant de 1 à 11 atomes de carbone éventuellement ramifiée,
- si B' est H, B est une chaine alkyle comportant de 3 à 11 atomes de carbone porteuse d'au moins un radical alkyle ramifié de formule CₙH₂ₙ₊₁ avec n compris dans la gamme de 1 à 5;
ledit procédé comprenant la métathèse en présence d'un catalyseur de métathèse entre :
- d'une part un **composé gras insaturé linéaire de formule I :**

   R-(CH₂)ₐ - [(CH=CH) -(CH₂)]_{b} - (CH₂)_{c} - R'

   dans laquelle
   R' est un radical -COOH, -COOR₁ ou -CN, R₁ étant le reste d'un mono-alcool ou d'un polyol, tel que diol ou triol,
   R est H ou une fonction acide, ester de mono-alcool ou nitrile et,
   a, b, c sont des entiers tels que 0 ≤ a ≤ 11 ; 1 ≤ b ≤ 6 ; 2 ≤ c ≤ 12, la somme a + 3*b + c + 1 étant comprise dans la gamme de 8 à 36, de préférence de 8 à 24, de préférence de 10 à 22, et de façon encore plus préférée de 10 à 18 ; et,
- d'autre part une **oléfine ramifiée II** comprenant de 4 à 23 atomes de carbone, dans laquelle la chaîne principale comporte au moins une « ramification » (ou « radical alkyle ») de formule CₙH₂ₙ₊₁ avec n compris dans la gamme de 1 à 5 lorsque (dans la formule III) B' est H.

### DESCRIPTION DE MODES DE REALISATION DE L'INVENTION

L'invention est maintenant décrite plus en détail et de façon non limitative dans la description qui suit.

Le **composé gras insaturé linéaire I** (parfois nommé aussi « molécule grasse » dans la présente description) utilisable dans le procédé de l'invention est avantageusement choisi parmi : les acides gras et esters d'acides gras, mono ou poly-insaturés, et leurs dérivés nitriles, issus des matières végétales ou animales, y compris les mono-, di-, et triglycérides qui en sont l'origine. A titre d'exemples, on peut citer les acides et les esters d'acides choisis parmi les acides : oléique, pétrosélénique, linoléique, ricinoléique, gadoléique, gondoïque, vaccénique, linolénique, palmitoléique, érucique, nervonique, etc, les triglycérides dont ils sont issus, et leurs mélanges.

Les composés gras insaturés utilisables dans le procédé de l'invention comprennent également les dérivés obtenus pas transformation chimique des molécules précédentes, tels que les nitriles obtenus par nitrilation de la fonction ester ou acide. Les composés gras insaturés comprennent également les molécules bi-fonctionnelles portant des fonctions acide, ester ou nitrile à chaque extrémité de la molécule (en général deux fonctions identiques), ainsi que les mono-, di- et triglycérides eux-mêmes éventuellement soumis à une réaction chimique préalable, telle que la méthanolyse, l'éthanolyse ou la métathèse avec une oléfine légère en C2-C4.

En particulier, les composés gras insaturés peuvent découler d'une transformation chimique des acides/esters gras/triglycérides cités ci-dessus par exemple par une métathèse croisée de ces derniers avec une oléfine légère, éthylène, propylène ou butènes conduisant à des molécules fonctionnelles comportant de 8 à 36, de préférence de 8 à 26, de préférence de 8 à 24, de préférence de 10 à 22, et de manière encore plus préférée de 10 à 18 atomes de carbone, et de préférence ω, ω-1 ou ω-2 insaturées, bien adaptées à la réaction de métathèse avec l'oléfine Il ramifiée.

De préférence, le procédé de métathèse de l'invention est mis en oeuvre à partir d'un composé gras insaturé comportant une fonction, et de préférence une seule fonction, de type ester de mono-alcool, ester de polyol ou de type nitrile. Comme décrit précédemment, les sources principales de ces composés sont les huiles ou les graisses naturelles d'origine animale ou végétale, y compris les algues, dont on peut extraire les triglycérides, et donc les éléments constitutifs de ces derniers.

Les esters gras insaturés de mono-alcools sont obtenus par exemple par action d'un alcool léger, méthanol ou éthanol de préférence, sur le triglycéride. Ainsi, selon un mode de réalisation avantageux du procédé de l'invention, un ester de mono-alcool obtenu par méthanolyse ou éthanolyse d'un triglycéride, est soumis à une réaction de métathèse croisée avec une oléfine branchée.

Les esters gras de polyols comprennent généralement des triglycérides extraits des huiles végétales, huiles ou graisses animales ou provenant des micro-algues. Ces esters gras sont, préalablement à la métathèse avec l'oléfine branchée, soumis à une étape préliminaire de métathèse croisée avec une (ou des) oléfine(s) légère(s), éthylène, propylène ou butène-1 ou butène-2, ou pentène-2, hexène-2, hexène-3, conduisant à la formation d'esters gras insaturés ayant une double liaison localisée à proximité du bout de la chaîne alkyle (esters de préférence ω, ω-1 ou ω-2 insaturés). Pour cette étape préliminaire on peut utiliser un mélange de ces oléfines légères. Cette étape préliminaire permet d'améliorer les performances de la métathèse croisée avec l'oléfine branchée grâce à la formation, au cours de cette réaction, d'oléfines légères gazeuses dont l'extraction du milieu réactionnel permet de déplacer l'équilibre de la réaction de métathèse.

Les nitriles gras insaturés sont généralement obtenus à partir des acides ou des esters gras correspondants par action de l'ammoniac puis déshydratation du composé, sel d'ammonium ou amide, formé selon des mécanismes réactionnels bien connus.

Dans une variante de mise en oeuvre du procédé de l'invention le composé I est un diester ou un dinitrile (par exemple en C18 ou C20) obtenu soit par homo-métathèse de l'ester ou du nitrile, produit principal de l'homo-métathèse, soit au cours d'une réaction de métathèse croisée au cours de laquelle le diester ou le dinitrile se forme comme coproduit. Dans ce cas la réaction de métathèse selon le procédé de l'invention génère au moins un composé ramifié et un composé qui est un ester ou nitrile plus court pouvant être à nouveau utilisé pour d'autres applications y compris une étape de métathèse.

**L'oléfine ramifiée II** utilisée pour la réaction de métathèse contient de préférence au moins 5 atomes de carbone, de manière plus préférée au moins 6 atomes de carbone, de manière encore plus préférée au moins 7 atomes de carbone, ou mieux au moins 8 atomes de carbone. L'oléfine comporte de préférence de 7 à 12, de préférence de 8 à 12 atomes de carbone au total.

L'oléfine utilisée pour la réaction de métathèse est soit mono-insaturée, soit polyinsaturée. Dans ce dernier cas les doubles liaisons sont de préférence non conjuguées. En effet, la présence de conjugaisons à tendance à désactiver le catalyseur de métathèse.

L'oléfine est de préférence mono-insaturée. En effet, lorsqu'on utilise des oléfines polyinsaturées, on observe une surconsommation de catalyseur et d'oléfines, et on est donc obligé de travailler à faible conversion pour limiter les pertes de produits, ce qui complique le procédé.

Avantageusement, l'oléfine ramifiée II est de formule : RₒR₀'C=CBB' dans laquelle :
B' est H ou une chaine alkyle comportant de 1 à 10 atomes de carbone et,
   - si B' est une chaine alkyle, B est une chaine alkyle comportant de 1 à 11 atomes de carbone éventuellement ramifiée ;
   - si B' est H, B est une chaine alkyle comportant de 3 à 11 atomes de carbone dont au moins un atome de carbone est porteur d'un radical alkyle de formule CₙH₂ₙ₊₁ avec n compris dans la gamme de 1 à 5, et
R'₀ est H ou une chaine alkyle comportant de 1 à 10 atomes de carbone et,
   - si R'₀ est H, Rₒ est H, CH₃, C₂H₅ ou une chaine alkyle comportant de 3 à 11 atomes de carbone dont au moins un atome de carbone est porteur d'un radical alkyle de formule CₙH₂ₙ₊₁ avec n compris dans la gamme de 1 à 5, et
   - si R'₀ est une chaine alkyle, R₀ est H ou une chaine alkyle comportant de 1 à 11 atomes de carbone éventuellement ramifiée.

Selon un mode de réalisation avantageux du procédé de l'invention, -B est une chaine alkyle de formule -(CR₂R₃)ₘ-CH₃ dans laquelle R₂ et R₃ répondent aux définitions données ci-après. La position du groupement -CH₃ à l'extrémité de cette chaine alkyle B (opposée à la double liaison de la formule II) est dans la présente description désignée par « ω », quel que soit le nombre d'atomes de carbone m le reliant à la double liaison.

Avantageusement, -R₀ est une chaine alkyle de formule -(CR₂'R₃')ₚ-CH₃ dans laquelle R₂' et R₃' répondent aux définitions données ci-après pour R₂ et R₃. De même, la position du groupement -CH₃ à l'extrémité de la chaine Rₒ (opposée à la double liaison de la formule II) est également désignée par « ω » dans la présente description de l'invention.

Selon un mode de réalisation préféré du procédé de l'invention, dans la formule II de l'oléfine, B' est H et -B est de formule -(CR₂R₃)ₘ-CH₃ dans laquelle R₂ et R₃ identiques ou différents, sont soit H, soit un radical alkyle saturé comportant de 1 à 5 atomes de carbone, les R₂ d'une part et les R₃ d'autre part reliés aux atomes de carbone de la chaine étant entre eux identiques ou différents, et l'un au moins des R₂ et R₃ étant un radical alkyle saturé, ledit au moins un carbone porteur dudit radical étant dans la chaine de préférence en position ω-i, i étant > 1 (i entier strictement supérieur à 1), notamment en position ω-2, ω-3, etc.

Avantageusement, la (ou les) ramification(s) de l'oléfine est (sont) localisée(s) de préférence plus proche(s) de la double liaison C=C que de l'extrémité de la chaine qui porte la(les)dite(s) ramification(s). En effet, il s'avère que le point de fusion des composés gras insaturés ramifiés ainsi obtenus par les procédé de l'invention est d'autant plus bas que la position d'une ramification s'éloigne de l'extrémité de chaine et se rapproche donc de la double liaison C=C.

Selon un mode de réalisation préféré du procédé de l'invention, chaque atome de carbone de la double liaison de la formule II : RₒR₀'C=CBB' est porteur d'une seule chaine alkyle. Autrement dit, et de manière préférée, les radicaux alkyles de ramification de l'oléfine ne sont pas liés à un atome de carbone d'une double liaison. Dans ces conditions, la réaction de métathèse se passe mieux, on observe une meilleure conversion, et moins de consommation de catalyseur.

De plus, dans l'oléfine II utilisée, les atomes de carbone portant la double liaison sont de préférence reliés d'un côté de la double liaison à une chaine d'au plus 2 autres atomes de carbone et sont donc de préférence du type alcène-1, alcène-2 ou alcène-3, ce qui facilite également la réaction de métathèse croisée selon l'invention. Le coproduit formé dans ce cas particulier du procédé de l'invention est un « léger » comprenant moins de 8 carbones, de préférence moins de 7, voire moins de 5, ou mieux moins de 3 carbones, facile à extraire du milieu réactionnel, et permettant ainsi de faire avancer plus vite la réaction de métathèse.

Suivant le degré de pureté et le degré de contrôle des propriétés physicochimiques du composé III ciblé, une oléfine branchée conforme à la définition ci-dessus peut être utilisée soit seule (et de préférence la plus pure possible), soit en mélange avec une ou plusieurs autres oléfines conformes ou non à la définition ci-dessus. A titre d'exemple, l'oléfine branchée peut être utilisée sous la forme d'un mélange avec une ou plusieurs oléfine(s) linéaire(s). Des exemples de mélanges d'oléfines appropriés pour le procédé de l'invention et commercialement disponibles, sont notamment les oligomères de propylène et de butène ou les mélanges d'oléfines issues de coupes pétrolières, par exemple les essences légères.

Le tableau 1 suivant fournit des exemples d'oléfines préférées dans lesquelles la double liaison ne porte pas de ramification. Seules les oléfines de 5 à 9 atomes de carbone y sont illustrées, afin de ne pas surcharger inutilement la description. Bien entendu la définition de l'oléfine ne se limite pas à ces exemples.

La première colonne indique la longueur de la chaîne linéaire principale (la plus longue) de l'oléfine, la seconde indique la position de la double liaison, les colonnes suivantes indiquent les positions des groupements méthyle, éthyle et propyle, et la dernière colonne le nombre total d'atomes de carbone de l'oléfine.

**Tableau 1**

| Longueur de Chaine | Position C=C | Position Méthyl | | | | Ethyl | Propyl | Nombre de Carbones |
|---|---|---|---|---|---|---|---|---|
| 4 | 1 | 3 | | | | | | 5 |
| 4 | 1 | 3 | 3 | | | | | 6 |
| 5 | 1 | 4 | | | | | | 6 |
| 5 | 1 | 3 | | | | | | 6 |
| 5 | 2 | 4 | | | | | | 6 |
| 5 | 1 | 4 | 4 | | | | | 7 |
| 5 | 1 | 3 | 3 | | | | | 7 |
| 5 | 1 | | | | | 3 | | 7 |
| 5 | 1 | 3 | 4 | | | | | 7 |
| 5 | 2 | 4 | 4 | | | | | 7 |
| 6 | 1 | 5 | | | | | | 7 |
| 6 | 1 | 4 | | | | | | 7 |
| 6 | 1 | 3 | | | | | | 7 |
| 6 | 2 | 4 | | | | | | 7 |
| 6 | 2 | 5 | | | | | | 7 |
| 6 | 3 | 2 | | | | | | 7 |
| 5 | 1 | 4 | | | | 3 | | 8 |
| 5 | 1 | 3 | 4 | 4 | | | | 8 |
| 5 | 1 | 3 | | | | 3 | | 8 |
| 5 | 1 | 3 | 3 | 4 | | | | 8 |
| 6 | 1 | 3 | 3 | | | | | 8 |
| 6 | 1 | 3 | 4 | | | | | 8 |
| 6 | 1 | 3 | 5 | | | | | 8 |
| 6 | 1 | 4 | 4 | | | | | 8 |
| 6 | 1 | 4 | 5 | | | | | 8 |
| 6 | 1 | 5 | 5 | | | | | 8 |
| 6 | 1 | | | | | 3 | | 8 |
| 6 | 1 | | | | | 4 | | 8 |
| 6 | 2 | 4 | 4 | | | | | 8 |
| 6 | 2 | 4 | 5 | | | | | 8 |
| 6 | 2 | 5 | 5 | | | | | 8 |
| 6 | 2 | | | | | 4 | | 8 |
| 6 | 3 | 2 | 2 | | | | | 8 |
| 6 | 3 | 2 | 5 | | | | | 8 |
| 7 | 1 | 3 | | | | | | 8 |
| 7 | 1 | 4 | | | | | | 8 |
| 7 | 1 | 5 | | | | | | 8 |
| 7 | 1 | 6 | | | | | | 8 |
| 7 | 2 | 4 | | | | | | 8 |
| 7 | 2 | 5 | | | | | | 8 |
| 7 | 2 | 6 | | | | | | 8 |
| 7 | 3 | 2 | | | | | | 8 |
| 7 | 3 | 5 | | | | | | 8 |
| 7 | 3 | 6 | | | | | | 8 |
| 5 | 1 | 3 | 3 | 4 | 4 | | | 9 |
| 5 | 1 | 3 | | 4 | | 3 | | 9 |
| 6 | 1 | 3 | 3 | 4 | | | | 9 |
| 6 | 1 | 3 | 3 | 5 | | | | 9 |
| 6 | 1 | 3 | 4 | 5 | | | | 9 |
| 6 | 1 | 3 | 4 | 4 | | | | 9 |
| 6 | 1 | 3 | 5 | 5 | | | | 9 |
| 6 | 1 | 4 | 4 | 5 | | | | 9 |
| 6 | 1 | 4 | 5 | 5 | | | | 9 |
| 6 | 1 | 3 | | | | 3 | | 9 |
| 6 | 1 | 3 | | | | 4 | | 9 |
| 6 | 1 | 4 | | | | 3 | | 9 |
| 6 | 1 | 4 | | | | 4 | | 9 |
| 6 | 1 | 5 | | | | 3 | | 9 |
| 6 | 1 | 5 | | | | 4 | | 9 |
| 6 | 1 | | | | | | 3 | 9 |
| 6 | 2 | 4 | 5 | 5 | | | | 9 |
| 6 | 2 | 4 | 4 | 5 | | | | 9 |
| 6 | 2 | 4 | | | | 4 | | 9 |
| 6 | 3 | 2 | 2 | 5 | | | | 9 |
| 7 | 1 | 3 | 3 | | | | | 9 |
| 7 | 1 | 3 | 4 | | | | | 9 |
| 7 | 1 | 3 | 5 | | | | | 9 |
| 7 | 1 | 3 | 6 | | | | | 9 |
| 7 | 1 | 4 | 4 | | | | | 9 |
| 7 | 1 | 4 | 5 | | | | | 9 |
| 7 | 1 | 4 | 6 | | | | | 9 |
| 7 | 1 | | | | | 3 | | 9 |
| 7 | 1 | | | | | 4 | | 9 |
| 7 | 1 | | | | | 5 | | 9 |
| 7 | 2 | 4 | 4 | | | | | 9 |
| 7 | 2 | 4 | 5 | | | | | 9 |
| 7 | 2 | 4 | 6 | | | | | 9 |
| 7 | 2 | 5 | 5 | | | | | 9 |
| 7 | 2 | 5 | 6 | | | | | 9 |
| 7 | 2 | 6 | 6 | | | | | 9 |
| 7 | 2 | | | | | 4 | | 9 |
| 7 | 2 | | | | | 5 | | 9 |
| 7 | 3 | 2 | 2 | | | | | 9 |
| 7 | 3 | 2 | 5 | | | | | 9 |
| 7 | 3 | 2 | 6 | | | | | 9 |
| 7 | 3 | 5 | 5 | | | | | 9 |
| 7 | 3 | 5 | 6 | | | | | 9 |
| 7 | 3 | 6 | 6 | | | | | 9 |
| 8 | 1 | 3 | | | | | | 9 |
| 8 | 1 | 4 | | | | | | 9 |
| 8 | 1 | 5 | | | | | | 9 |
| 8 | 1 | 6 | | | | | | 9 |
| 8 | 1 | 7 | | | | | | 9 |
| 8 | 2 | 4 | | | | | | 9 |
| 8 | 2 | 5 | | | | | | 9 |
| 8 | 2 | 6 | | | | | | 9 |
| 8 | 2 | 7 | | | | | | 9 |
| 8 | 3 | 2 | | | | | | 9 |
| 8 | 3 | 5 | | | | | | 9 |
| 8 | 3 | 6 | | | | | | 9 |
| 8 | 3 | 7 | | | | | | 9 |
| 8 | 4 | 2 | | | | | | 9 |
| 8 | 4 | 3 | | | | | | 9 |

La **réaction de métathèse croisée** selon le procédé de l'invention est conduite en présence d'au moins un catalyseur de métathèse.

Il existe de nombreux catalyseurs de métathèse, et ils sont bien connus. On peut citer par exemple les complexes au tungstène développés par Schrock et al (J. Am. Chem. Soc. 108:2771, 1986) ou Basset et al. (Angew. Chem., Ed. Engl. 31:628, 1992). Plus récemment, sont apparus les catalyseurs dits de Grubbs (voir Grubbs et al., Angew. Chem., Ed. Engl. 34 :2039, 1995 et Organic Letters 1:953, 1999) qui sont des complexes ruthénium-benzylidène opérant en catalyse homogène. D'autres travaux ont été conduits pour la réalisation de catalyseurs immobilisés, c'est-à-dire de catalyseurs dont le principe actif est celui du catalyseur homogène, notamment les complexes ruthénium-carbène, immobilisés sur un support inactif.

Le procédé selon l'invention utilise avantageusement un catalyseur de métathèse de type ruthénium-carbène.

Les catalyseurs ruthénium-carbène sont choisis de préférence parmi les catalyseurs chargés ou non-chargés de formule générale :

(X₁)ₐ (X₂)_{b}Ru(carbène C) (L₁)_{c}(L₂)_{d}(L₃)ₑ

dans laquelle :
- a, b, c, d et e sont des nombres entiers, identiques ou différents, avec a et b égaux à 0, 1 ou 2 ; c,d et e égaux à 0, 1, 2, 3 ou 4;
- X₁ et X₂, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et dérivés ; X₁ ou X₂ peuvent être liés à L₁ ou L₂ ou au carbène C de façon à former un ligand bidenté ou chélate sur le ruthénium ; et
- L₁, L₂ et L₃ identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéther, ou un carbène hétérocyclique ; L₁, L₂ ou L₃ peuvent être liés au carbène C de façon à former un ligand bidenté ou chélate, ou tridenté.

Le carbène C est représenté par la formule générale : CR₁R₂ pour laquelle R₁ et R₂ sont des groupes identiques ou différents tels que l'hydrogène ou tout autre groupe hydrocarboné, fonctionnalisé ou non, de type saturé, insaturé, cyclique, aromatique, branché et/ou linéaire. A titre d'exemples, on pourra citer les complexes du ruthénium alkylidènes, benzylidène, benzylidène éther, ou cumylènes tels que les vinylidènes Ru=C=CHR ou allénylidènes Ru=C=C=CR₁R₂ ou indénylidènes.

Un groupe fonctionnel (permettant d'améliorer la rétention du complexe du ruthénium dans un liquide ionique) peut être greffé sur au moins l'un des ligands X₁, X₂, L₁, L₂, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

Le catalyseur de métathèse peut être éventuellement hétérogénéisé sur un support afin de faciliter sa récupération/recyclage.

Les catalyseurs de métathèse croisée du procédé de l'invention sont de préférence des carbènes du ruthénium décrits par exemple dans Aldrichimica Acta, vol 40, n°2, 2007, p.45-52.

Des exemples de tels catalyseurs sont les catalyseurs de Grubbs, les catalyseurs Hoveyda-Grubbs, les catalyseurs Piers-Grubbs, et autres catalyseurs de métathèse du même type, qu'ils soient dits de « 1^{ère} génération », « 2^{ème} génération » ou de « 3^{ème} génération ».

Les catalyseurs de Grubbs sont basés sur un atome de ruthénium entouré par 5 ligands :
- 2 ligands anioniques, tels que des halogénures ;
- 2 ligands donneurs d'électrons, tels que les tri-alkyl-phosphines, ou les carbènes N-hétérocycliques saturés (appelés ligands NHC) ;
- un groupement alkylidène, tel que des groupements méthylènes =CR₂ substitués ou non.

On classe ces catalyseurs de métathèse en deux catégories, suivant la nature de leurs ligands L donneurs d'électrons :
- ceux qui contiennent deux ligands phosphine (et pas de ligand NHC saturé), développés en premier, sont des catalyseurs de type 1^{ère} génération ;
- ceux qui contiennent un ligand NHC saturé (carbène hétérocyclique) sont des catalyseurs de type 2^{ème} génération.

Un type de catalyseur dit « Hoveyda-Grubbs » contient parmi les ligands donneurs d'électrons, un ligand chélatant benzylidène-éther, et soit une phosphine (1^{ère} génération) soit un ligand NHC saturé (2^{ème} génération), le plus souvent substitué par des phényles généralement substitués par des groupements mésityles (Mes) ou bien par des groupements isopropyles (iPr).

Un autre type de catalyseur dit « Piers-Grubbs », forme un complexe cationique à quatre ligands qui ne nécessite pas la dissociation d'un ligand avant la réaction.

D'autres types de catalyseurs sont les catalyseurs « Umicore », « Zanan », « Grela ».

De manière générale, le choix du catalyseur dépend de la réaction considérée.

De préférence, le catalyseur utilisé dans le procédé de l'invention est dépourvu de phosphine.

A titre d'exemple, des catalyseurs particulièrement adaptés pour le procédé de l'invention sont les catalyseurs suivants :
(1) Le catalyseur désigné par « Hoveyda-Grubbs 2 », de formule suivante : 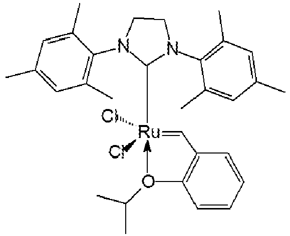
(2) Le catalyseur désigné par « M51 », de formule suivante : 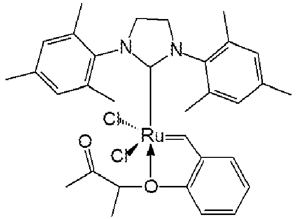
(3) Le catalyseur désigné par « M71-SIPr », de formule suivante : 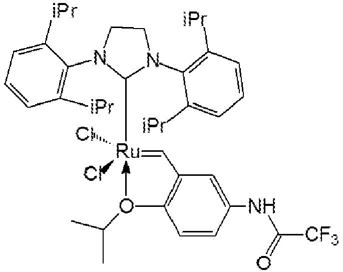
(4) Le catalyseur désigné par « M71-SIMes », de formule suivante : 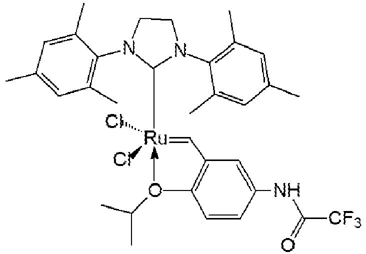
(5) Le catalyseur désigné par « M72-SIPr », de formule suivante : 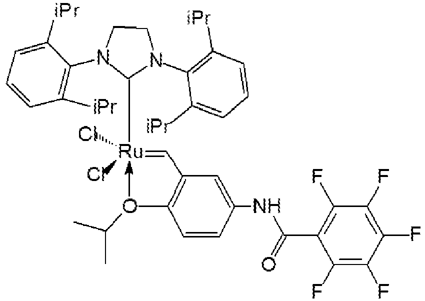
(6) Le catalyseur désigné par « M73-SIPr », de formule suivante : 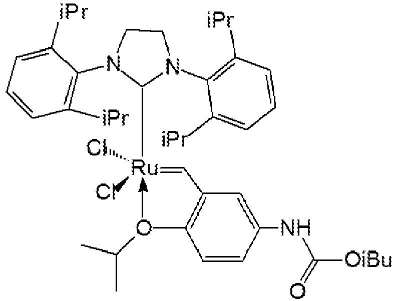
(7) Le catalyseur désigné par « M74-SIPr », de formule suivante : 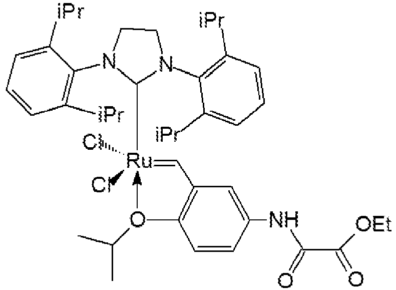
(8) Le catalyseur désigné par « Nitro-Grela-SIMes », de formule suivante : 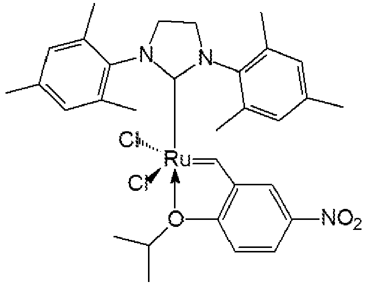
(9) Le catalyseur désigné par « Nitro-Grela-SIPr », de formule suivante : 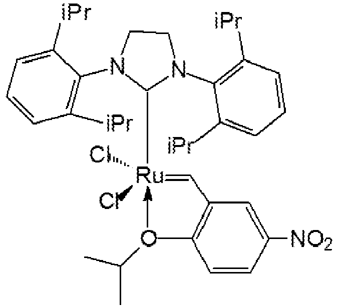
(10) Le catalyseur désigné par « Apeiron AS2034 », de formule suivante : 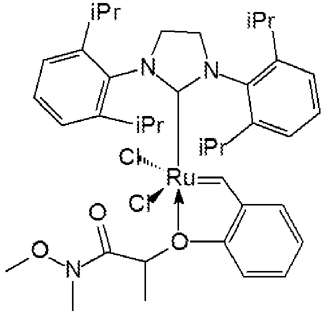
(11) Le catalyseur désigné par « Zannan 44-0082 (Strem) », de formule suivante : 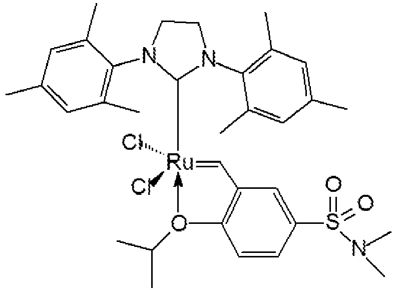
(12) Le catalyseur désigné par « M831-SIPr », de formule suivante : 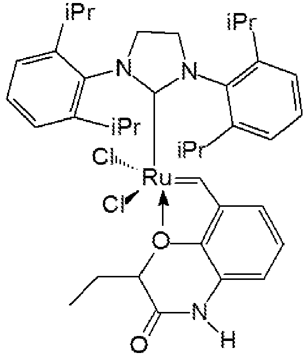
(13) Le catalyseur désigné par « M832-SIPr », de formule suivante : 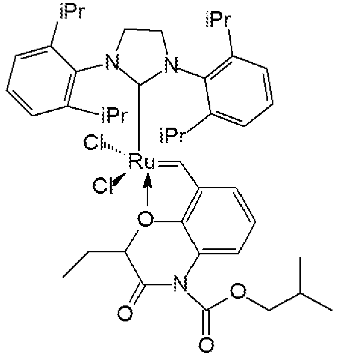
(14) Le catalyseur désigné par « M853-SIPr », de formule suivante : 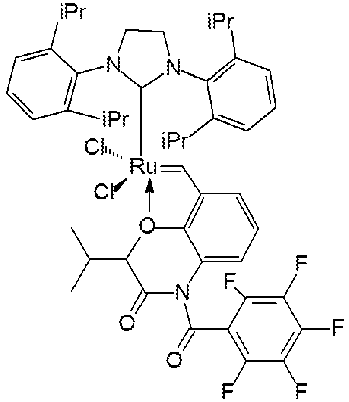
(15) Le catalyseur désigné par « M863-SIPr », de formule suivante : 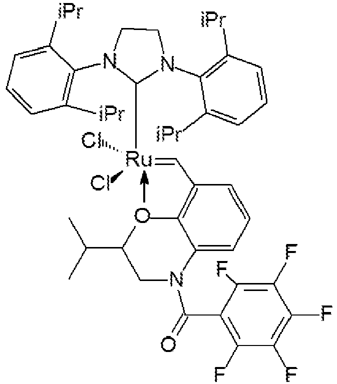
(14) Le catalyseur désigné par « Materia C711 », de formule suivante :

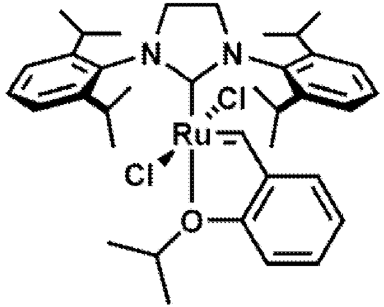

De préférence, la réaction de métathèse est conduite en milieu liquide et dans les conditions opératoires suivantes.

La température de la réaction est généralement comprise dans la gamme de 20 à 160 °C, et de préférence dans la gamme de 20 à 120 °C.

La pression de la réaction est généralement comprise dans la gamme de 1 à 30 bars. La réaction est de préférence conduite à basse pression comprise dans la gamme de 1 à 10 bars et de façon plus préférée à la pression atmosphérique lorsque la température d'ébullition des réactifs mis en jeu le permet. En effet, si l'on vise toujours un dégagement d'une oléfine légère que ce soit de l'éthylène ou une autre, il est avantageux de travailler à basse pression, pression atmosphérique de préférence. Cependant, il existe des contraintes liées au point d'ébullition de l'oléfine utilisée dans le procédé de l'invention qui est conduit en phase liquide. Dans le cas où ce point d'ébullition est bas, par exemple pour une oléfine en C5 où celui-ci est de l'ordre de 20 °C, il est nécessaire de travailler sous pression. Cette contrainte disparaît avec les oléfines supérieures. Par exemple, une oléfine en C7, dont le point de d'ébullition est de l'ordre de 80 °C, permet de travailler à la pression atmosphérique.

La réaction peut être conduite sans solvant ou en présence d'un solvant comme le toluène, les xylènes ou le dichlorométhane par exemple. La réaction est de préférence conduite sans solvant.

Dans un mode de réalisation préféré du procédé de l'invention, on ajoute au milieu réactionnel le catalyseur et/ou l'oléfine (II) et/ou la molécule grasse (composé I) en continu pendant le processus réactionnel.

**Le composé gras insaturé ramifié III** issu de la réaction de métathèse croisée du procédé de l'invention, peut servir de matière première à toute une gamme de réactions. On peut citer à titre d'exemples non limitatifs les réactions ci-après :
- hydrogénation de ladite au moins une double liaison C=C (du composé III ou issue du composé III), qui conduit selon le type d'hydrogénation pour les esters à des esters saturés, des alcools saturés, voire des paraffines branchées, et pour les nitriles à des amines ramifiées ;
- hydrolyse d'ester qui conduit à un acide ;
- nitrilation d'acide ou d'ester, selon que l'on ait intermédiairement une hydrolyse ou pas, puis conversion éventuelle du nitrile en amine par hydrogénation ;
- époxydation de ladite au moins une double liaison C=C (du composé III ou issue du composé III), avec ou sans ouverture du cycle époxy, pour former des diols, carbonates, esters..., conduisant à l'époxy d'une chaine ramifiée, qui confère des propriétés de viscosité améliorées. Dans ce cas, il est avantageux de travailler avec des composés polyinsaturés ;
- nouvelle étape de métathèse en particulier dans le cas où la métathèse avec oléfine branchée a été faite sur un diester. Dans ce cas, la nouvelle métathèse permet de recycler une partie du coproduit de la réaction qui est un ester court, par exemple du décénoate de méthyle ou de l'undécénoate de méthyle.

En fonction des applications visées, certaines chaines grasses sont plus appropriées que d'autres, ce qui implique une sélection des huiles végétales, mais aussi de la nature de la matière première utilisée pour la réaction de métathèse, à savoir triglycéride, ester de monoalcool ou polyalcool, acide ou nitrile.

Les huiles végétales et graisses animales peuvent être regroupées selon leurs teneurs en acides gras saturés, monoinsaturés, et polyinsaturés.
Les huiles riches en chaines grasses polyinsaturées incluent les huiles de carthame (haut linoléique), de lin, de tournesol, de noix et de noisette, de soja, de coton, de maïs, de Jatropha, de colza, de cameline.
Les huiles riches en chaines grasses saturées ne conviennent en général pas pour la réaction de métathèse, qui nécessite des insaturations pour que la réaction puisse s'effectuer. Cependant, ces huiles sont en général bon marché et contiennent par contre très peu de chaines polyinsaturées, ce qui présente l'avantage d'obtenir des réactions beaucoup plus sélectives. On trouve dans ce groupe d'huiles végétales les huiles de palme, et les graisses animales telles que le gras de boeuf et le lard. En revanche, les huiles de babassu, de noix de coco, de palmiste ne sont pas intéressantes non seulement en raison de leur plus faible teneur en insaturations mais aussi de leur prix beaucoup plus élevé.
Le dernier groupe d'huiles végétales est constitué des huiles riches en chaines mono-insaturées, notamment des huiles dites oléiques. C'est le cas des huiles de colza, notamment le colza oléique et haut oléique (à plus forte teneur en acide oléique), du tournesol oléique, du crambe, du colza érucique, du carthame oléique, de la lunaire, de l'huile d'olive, du ricin, de lesquerella notamment des variétés fendleri, grandi flora et gordonii.

Selon un mode de réalisation particulier, le procédé de l'invention comprend en outre une étape d'hydrogénation pour la synthèse de composés gras ramifiés saturés, notamment d'ester saturés, d'alcools saturés, de nitriles saturés, d'amines saturées, d'acides gras saturés. Dans ce cas, le composé I utilisé est de préférence un ester gras mono insaturé, issu d'huile(s) riche(s) en acides gras mono-insaturés, ou bien d'ester(s) gras ayant déjà subi une étape de métathèse croisée avec une oléfine légère ou une étape de craquage thermique.
Selon un autre mode de réalisation, le procédé de l'invention est utilisé pour la synthèse de composés gras insaturés ramifiés utilisés comme lubrifiants. Dans ce dernier cas, le composé I (aussi appelé « molécule grasse ») est choisi de préférence parmi : des triglycérides, notamment des triglycérides ayant déjà subi une étape de métathèse ou des esters d'acide gras mono-insaturés notamment des esters d'acide gras ω-insaturés. Selon encore un autre mode de réalisation, le procédé de l'invention est utilisé pour la synthèse de composés gras insaturés ramifiés devant subir une époxydation. Dans ce cas le composé I (ou molécule grasse) est avantageusement choisi parmi les triglycérides et les esters issus des huiles riches en acides gras polyinsaturés.
Selon un autre mode de réalisation, le procédé de l'invention est utilisé pour la fabrication de fluides diélectriques. Dans ce cas le composé I (ou molécule grasse) est avantageusement choisi parmi les triglycérides et les esters issues des huiles riches en acides gras mono-insaturés et de préférence ayant une insaturation en position delta-5 à delta-9.
De préférence le produit obtenu selon le procédé de l'invention est au moins partiellement hydrogéné, afin d'augmenter la stabilité des formulations qui le comprennent.
La présente invention a également pour objet l'utilisation du produit obtenu selon le procédé de l'invention pour la fabrication d'au moins un des produits suivants : les fluides diélectriques, les tensioactiactifs de spécialité, les émulsifiants, les agents de friction, les additifs antistatiques, additifs anti-buée, les agents de démoulage, les dispersants de pigments, les lubrifiants haute performance, les cires et émulsifiants de cires, les additifs de transformation de polymères, les stabilisants de PVC, les encres, résines, peintures, vernis, les solvants, les rouges à lèvres, les crèmes pour la peau, les déodorants, notamment sous forme de sticks, les colorants pour cheveux, les shampoings et autres savons liquides, les mousses à raser, les lessives, les nettoyants, les adoucissants textiles, et leurs mélanges. Et de manière plus générale, le produit obtenu selon le procédé de l'invention peut être utilisé dans toutes les applications exigeantes en termes de pureté, de bas point de fusion, et de stabilité (ou résistance) à l'oxydation.

### EXEMPLES

Le(s) exemple(s) suivant(s) illustre(nt) l'invention sans la limiter.

### Exemple 1

L'essai est réalisé avec le dispositif expérimental suivant.
Le dispositif est composé d'un ballon de 600 ml muni d'une arrivée d'azote, d'une sonde de température, d'un réfrigérant (surmonté d'un robinet et sortant sur un bulleur), d'un bain d'huile et d'une entrée pour les réactifs et d'un pousse seringue porteur des seringues d'injection de l'oléfine et du catalyseur ajoutés en continu.

On purge tout d'abord le montage à l'azote dont l'alimentation sera coupée au moment du chargement de l'oléfine et du catalyseur.

On prépare le catalyseur M71SiPr d'Umicore comme suit : 10,2 mg, pesés dans un sabot en verre, sont dissous dans 21,6 g de toluène dans un tube de Schlenk sous azote. 5 ml de cette solution soit 2,02 mg de catalyseur sont introduits dans une première seringue.

On prépare une deuxième seringue dans laquelle on introduit 4,4 g de 5-méthyl-hexène-1 (Masse molaire : 98 g/mole) soit 44 mmoles.

On charge le réacteur avec 150 g de toluène puis ensuite avec 15 g de 10-undécénoate de méthyle (Masse molaire 198 g/mole) soit 76 mmoles purifiées par adsorption, 1 g de dodécane (utilisé comme étalon interne) et enfin avec 4,4 g d'oléfine (5-méthyl-hexène-1) soit à nouveau 44 mmoles.

On chauffe à 110°C en laissant ouvert le robinet du réfrigérant et on contrôle la température du milieu. (bain d'huile à 145°C)

On effectue un premier prélèvement à t=0 et on déclenche le début de la réaction (t=0) et on ajoute le catalyseur et l'oléfine (44 mmoles préparées ci-dessus) en continu pendant 2 heures à un débit de 22 mmoles/heure pour l'oléfine. On effectue un prélèvement à t= 120 min et on fait une analyse GC des échantillons. A la fin de la réaction on coupe la chauffe, le bain du réfrigérant et on laisse refroidir.

Les conditions d'analyse GC sont : Colonne HP5 de 30 m x 0.32 mm, épaisseur 0,25 µm, T Injecteur = 300 °C, T détecteur FID = 340 °C, Colonne 150 °C pendant 5 minutes, puis 10 °C/min jusqu'à 320 °C, puis 30 min à 320 °C.

La conversion de l'undécénoate de méthyle est de 85 %, et le rendement en composé gras insaturé ramifié (produit de métathèse croisée) 14-Méthyl-pentadec-10-énoate de méthyle - est de 71 %.

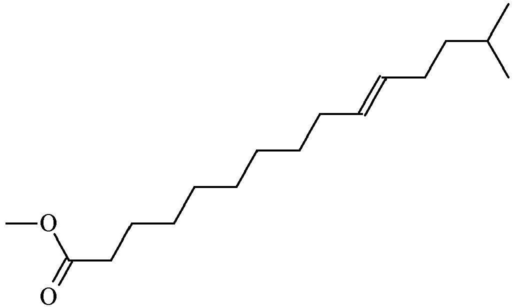

### Exemple 2 : Métathèse croisée décénoate de méthyle + néohexène

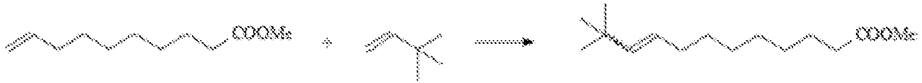

Dans un réacteur en verre de 250ml purgé à l'azote, on charge 10g de 9-décénoate de méthyle (54 mmol) purifié sur alumine, 18,1 g de 3,3-diméthyl-1-butène (neohexene, 215 mmol, 4 équivalents) et 100g de toluène. On chauffe à 40°C, puis on ajoute, sur une période de 2 heures, 3,4 mg de catalyseur M73-SiPr fourni par la société Umicore (75 ppm mol) dissous dans 5 ml de toluene. A la fin de l'ajout, le taux de conversion du décénoate de méthyle mesuré par CPG est de 86%. La sélectivité en produit de métathèse croisée est de 35%.

## Revendications

1. Procédé de synthèse de composé gras insaturé ramifié,
de formule III : A-CH=CB'-B dans laquelle
A est un radical constitué d'une chaîne alkyle linéaire, éventuellement insaturée, comprenant de 1 à 20 atomes de carbone et comportant une fonction terminale acide, ester (de mono-alcool ou de polyol), ou nitrile,
B' est H ou une chaine alkyle comportant de 1 à 10 atomes de carbone, et
B est :
- si B' est une chaine alkyle, B est une chaine alkyle comportant de 1 à 11 atomes de carbone éventuellement ramifiée,
- si B' est H, B est une chaine alkyle comportant de 3 à 11 atomes de carbone porteuse d'au moins un radical alkyle ramifié de formule CₙH₂ₙ₊₁ avec n compris dans la gamme de 1 à 5;
ledit procédé comprenant la métathèse en présence d'un catalyseur de métathèse entre :
- d'une part un composé gras insaturé linéaire de formule I :
R-(CH₂)ₐ-[(CH=CH)-(CH₂)]_{b}-(CH₂)_{c}-R'
dans laquelle
R' est un radical -COOH, -COOR₁ ou -CN, R₁ étant le reste d'un mono-alcool ou d'un polyol, tel que diol ou triol,
R est H ou une fonction acide, ester de mono-alcool ou nitrile et,
a, b, c sont des entiers tels que 0 ≤ a ≤ 11 ; 1 ≤ b ≤ 6 ; 2 ≤ c ≤ 12, la somme a + 3*b + c + 1 étant comprise dans la gamme de 8 à 36, de préférence de 8 à 24, de préférence de 10 à 22, et de façon encore plus préférée de 10 à 18 ; et,
- d'autre part une oléfine ramifiée II comprenant de 4 à 23 atomes de carbone, dans laquelle la chaîne principale comporte au moins une ramification de formule CₙH₂ₙ₊₁ avec n compris dans la gamme de 1 à 5 lorsque B' est H.

2. Procédé selon la revendication 1, dans lequel le composé gras insaturé linéaire I est choisi parmi : les acides gras, les esters d'acides gras, mono ou poly-insaturés, y compris les mono-, di-, et triglycérides, et les dérivés nitriles, des acides gras suivants : oléique, pétrosélénique, linoléique, ricinoléique, gadoléique, gondoïque, vaccénique, linolénique, palmitoléique, érucique, nervonique, et leurs mélanges.

3. Procédé selon l'une quelconque des revendications 1 ou 2, dans lequel le composé I est choisi parmi :
- les dérivés nitriles obtenus par nitrilation d'une fonction ester ou acide d'un ester ou acide gras,
- les molécules bi-fonctionnelles portant des fonctions acide, ester ou nitrile à chaque extrémité de la molécule,
- les triglycérides éventuellement soumis à une réaction chimique, telle que la méthanolyse, l'éthanolyse ou la métathèse avec une oléfine légère en C2-C4, ainsi que
- les molécules fonctionnelles comportant de 8 à 12 atomes de carbone et de préférence w, ω-1 ou ω-2 insaturées, issues de métathèse croisée d'acides/esters gras avec une oléfine légère, telle que éthylène, propylène ou butène.

4. Procédé selon l'une quelconque des revendications précédentes, dans lequel l'oléfine ramifiée II utilisée pour la réaction de métathèse contient de préférence au moins 5 atomes de carbone, et comporte de préférence de 7 à 12, de préférence de 8 à 12 atomes de carbone au total.

5. Procédé selon l'une quelconque des revendications précédentes, dans lequel l'oléfine ramifiée II est de formule : RₒR₀'C=CBB' dans laquelle :
B' est H ou une chaine alkyle comportant de 1 à 10 atomes de carbone et,
- si B' est une chaine alkyle, B est une chaine alkyle comportant de 1 à 11 atomes de carbone éventuellement ramifiée ;
- si B' est H, B est une chaine alkyle comportant de 3 à 11 atomes de carbone dont au moins un atome de carbone est porteur d'un radical alkyle de formule CₙH₂ₙ₊₁ avec n compris dans la gamme de 1 à 5, et
R'o est H ou une chaine alkyle comportant de 1 à 10 atomes de carbone et,
- si R'o est H, Rₒ est H, CH₃, C₂H₅ ou une chaine alkyle comportant de 3 à 11 atomes de carbone dont au moins un atome de carbone est porteur d'un radical alkyle de formule CₙH₂ₙ₊₁ avec n compris dans la gamme de 1 à 5, et
- si R'o est une chaine alkyle, R₀ est H ou une chaine alkyle comportant de 1 à 11 atomes de carbone éventuellement ramifiée.

6. Procédé selon la revendication 5, dans lequel dans la formule II de l'oléfine, B' est H et -B est de formule -(CR₂R₃)ₘ-CH₃ dans laquelle R₂ et R₃ identiques ou différents, sont soit H, soit un radical alkyle saturé comportant de 1 à 5 atomes de carbone, les R₂ d'une part et les R₃ d'autre part reliés aux atomes de carbone de la chaine étant entre eux identiques ou différents, et l'un au moins des R₂ et R₃ étant un radical alkyle saturé, le carbone porteur dudit radical étant dans la chaine de préférence en position ω-i, i étant > 1.

7. Procédé selon l'une quelconque des revendications précédentes, dans lequel ladite au moins une ramification de l'oléfine est localisée de préférence plus proche de la double liaison C=C que de l'extrémité de la chaine qui porte ladite ramification.

8. Procédé selon l'une quelconque des revendications 5 à 7, dans lequel chaque atome de carbone de la double liaison de la formule II : RₒR₀'C=CBB' est porteur d'une seule chaine alkyle.

9. Procédé selon l'une quelconque des revendications précédentes, dans lequel les atomes de carbone de ladite au moins une double liaison de l'oléfine sont reliés d'un côté de la double liaison à une chaine d'au plus 2 autres atomes de carbone, et sont de préférence du type alcène-1, alcène-2 ou alcène-3.

10. Procédé selon l'une quelconque des revendications précédentes, dans lequel on utilise au moins un catalyseur ruthénium-carbène choisis parmi les catalyseurs chargés ou non-chargés de formule générale :
(X₁)ₐ (X₂)_{b}Ru(carbène C) (L₁)_{c}(L₂)_{d} (L₃)ₑ
dans laquelle :
- a, b, c, d et e sont des nombres entiers, identiques ou différents, avec a et b égaux à 0, 1 ou 2 ; c,d et e égaux à 0, 1, 2, 3 ou 4;
- X₁ et X₂, identiques ou différents, représentent chacun un ligand mono- ou multi-chélatant, chargé ou non, tel que les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et dérivés, X₁ ou X₂ pouvant être liés à L₁ ou L₂ ou au carbène C de façon à former un ligand bidenté ou chélate sur le ruthénium ; et
- L₁, L₂ et L₃ identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéther, ou un carbène hétérocyclique, L₁, L₂ ou L₃ pouvant être liés au carbène C de façon à former un ligand bidenté ou chélate, ou tridenté.

11. Procédé selon la revendications 10 **caractérisé par** l'utilisation d'un catalyseur comprenant un ligand NHC saturé substitué par des phényles substitués par des groupements mésityle (Mes) ou isopropyle (iPr), de préférence l'utilisation du catalyseur de formule :

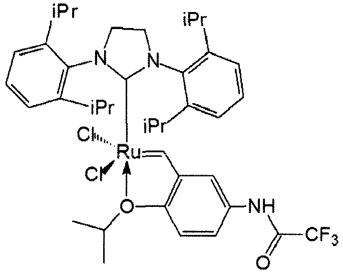

12. Procédé selon l'une quelconque des revendications précédentes **caractérisé en ce qu'**il est conduit en milieu liquide à une température comprise dans la gamme de 20 à 160 °C et sous une pression comprise dans la gamme de 1 à 30 bars.

13. Procédé selon la revendication 8 **caractérisé en ce qu'**il est conduit à une température comprise dans la gamme de 20 à 120 °C et sous une pression comprise dans la gamme de 1 à 10 bars.

14. Procédé selon l'une quelconque des revendications précédentes **caractérisé en ce que** la réaction est conduite en présence d'un solvant comme le toluène, les xylènes ou le dichlorométhane.

15. Procédé selon l'une quelconque des revendications précédentes **caractérisé en ce qu'**il comprend en outre, après l'obtention du composé III porteur d'au moins une double liaison C=C, au moins l'une des réactions suivantes :
• hydrogénation de ladite au moins une double liaison C=C, qui conduit selon le type d'hydrogénation pour les esters à des esters saturés, des alcools saturés, voire des paraffines branchées, et pour les nitriles à des amines ramifiées ;
• hydrolyse d'ester qui conduit à un acide ;
• nitrilation d'acide ou d'ester, selon que l'on ait intermédiairement une hydrolyse ou pas, puis conversion éventuelle du nitrile en amine par hydrogénation ;
• époxydation de ladite au moins une double liaison C=C, avec ou sans ouverture du cycle époxy, pour former des diols, carbonates, esters, conduisant à l'époxy d'une chaine ramifiée ; et/ou
• nouvelle étape de métathèse, en particulier dans le cas où la métathèse avec oléfine branchée a été faite sur un diester.

16. Utilisation du composé gras insaturé ramifié répondant à la formule III A-CH=CB'-B obtenu par le procédé selon l'une quelconque des revendications 1 à 14 pour la fabrication d'au moins un des produits suivants : les fluides diélectriques, les tensioactifs de spécialité, les émulsifiants, les agents de friction, les additifs antistatiques, additifs anti-buée, les agents de démoulage, les dispersants de pigments, les lubrifiants haute performance, les cires et émulsifiants de cires, les additifs de transformation de polymères, les stabilisants de PVC, les encres, résines, peintures, vernis, les solvants, les rouges à lèvres, les crèmes pour la peau, les déodorants, notamment sous forme de sticks, les colorants pour cheveux, les shampoings et autres savons liquides, les mousses à raser, les lessives, les nettoyants, les adoucissants textiles, et leurs mélanges.

## Patentansprüche

1. Verfahren zur Synthese einer fetten ungesättigten verzweigten Verbindung der Formel III: A-CH=CB'-B, wobei
A ein Rest ist, der aus einer linearen, eventuell ungesättigten Alkylkette besteht, die 1 bis 20 Kohlenstoffatome aufweist und eine Säure-, Ester- (eines Monoalkohols oder Polyols), oder Nitril-Endgruppe aufweist,
es sich bei B' um H oder um eine Alkylkette handelt, die 1 bis 10 Kohlenstoffatome aufweist, und es sich bei B handelt um:
falls es sich bei B' um eine Alkylkette handelt, es sich bei B um eine Alkylkette handelt, die 1 bis 11 eventuell verzweigte Kohlenstoffatome aufweist,
falls es sich bei B' um H handelt, es sich bei B um eine Alkylkette handelt, die 3 bis 11 Kohlenstoffatome aufweist und die mindestens einen verzweigten Alkylrest der Formel CₙH₂ₙ₊₁ trägt, wobei n im Bereich von 1 bis 5 liegt;
wobei das Verfahren eine Metathese unter Vorhandensein eines Metathese-Katalysators beinhaltet, und zwar zwischen:
einesteils einer fetten ungesättigten linearen Verbindung der Formel I:
R-(CH₂)ₐ-[(CH=CH)-(CH₂)]_{b}-(CH₂)_{c}-R',
wobei
R' ein -COOH, -COOR₁ oder -CN -Molekülrest ist, wobei R₁ der Rest eines Monoalkohols oder eines Polyols, etwa eines Diols oder eines Triols ist,
es sich bei R um H oder um eine Säure-, eine Monoalkohol-Ester- oder eine Nitril-Gruppe handelt, und
a, b, c Ganzzahlen sind, und zwar 0 ≤ a ≤ 11; 1 ≤ b ≤ 6; 2 ≤ c ≤ 12, wobei die Summe a + 3*b + c + 1 im Bereich von 8 bis 36, vorzugsweise von 8 bis 24, vorzugsweise 10 bis 22, und noch stärker bevorzugt von 10 bis 18 liegt; und
andernteils einem verzweigten Olefin II, das 4 bis 23 Kohlenstoffatome aufweist, bei dem die Hauptkette mindestens eine Verzweigung der Formel CₙH₂ₙ₊₁ aufweist, wobei n im Bereich von 1 bis 5 liegt, wenn es sich bei B' um H handelt.

2. Verfahren nach Anspruch 1, wobei die fette ungesättigte lineare Verbindung I gewählt ist aus: Fettsäuren, Fettsäureestern, und zwar mono- oder polyungesättigt, einschließlich Mono-, Di-und Triglyceriden, und Nitrilderivaten, der folgenden Fettsäuren: Ölsäure, Petroselinsäure, Linolsäure, Ricinolsäure, Gadoleinsäure, Gondosäure, Vaccensäure, Linolensäure, Palmitoleinsäure, Erucasäure, Nervonsäure, und Gemischen von diesen.

3. Verfahren nach einem der Ansprüche 1 oder 2, wobei die Verbindung I gewählt ist aus:
Nitrilderivaten, die durch Nitrilierung einer Ester- oder Säureester- oder Fettsäuregruppe erhalten werden,
zwei funktionelle Gruppen aufweisenden Molekülen, die eine Säure-, Ester- oder Nitrilgruppe an jedem Ende des Moleküls tragen,
Triglyceriden, die eventuell einer chemischen Reaktion unterzogen wurden, wie beispielsweise einer Methanolyse, einer Ethanolyse oder einer Metathese mit einem leichten C2-C4-Olefin, sowie
funktionellen Molekülen, die 8 bis 12 Kohlenstoffatome aufweisen und vorzugsweise ω, ω-1 oder ω-2 -ungesättigt sind, und zwar entstanden durch Kreuzmetathese von Fettsäuren/- estern mit einem leichten Olefin, wie beispielsweise Ethylen, Propylen oder Buten.

4. Verfahren nach einem der vorhergehenden Ansprüche, wobei das verzweigte Olefin II, das für die Metathese-Reaktion verwendet wird, vorzugsweise mindestens 5 Kohlenstoffatome enthält, und vorzugsweise 7 bis 12, und vorzugsweise 8 bis 12 Kohlenstoffatome insgesamt enthält.

5. Verfahren nach einem der vorhergehenden Ansprüche, wobei das verzweigte Olefin II die Formel hat: RoRo'C=CBB' , wobei:
es sich bei B' um H oder um eine Alkylkette handelt, die 1 bis 10 Kohlenstoffatome aufweist;
falls B' eine Alkylkette ist, es sich bei B um eine Alkylkette handelt, die 1 bis 11 eventuell verzweigte Kohlenstoffatome aufweist,
falls es sich bei B' um H handelt, B eine Alkylkette ist, die 3 bis 11 Kohlenstoffatome aufweist, von denen mindestens ein Kohlenstoffatom einen Alkylrest der Formel CₙH₂ₙ₊₁ trägt, wobei n im Bereich von 1 bis 5 liegt;
es sich bei R'o um H oder um eine Alkylkette handelt, die 1 bis 10 Kohlenstoffatome aufweist, und
falls es sich bei R'o um H handelt, es sich bei R₀ um H, CH₃, C₂H₅ oder um eine Alkylkette handelt, die 3 bis 11 Kohlenstoffatome aufweist, von denen mindestens ein Kohlenstoffatom einen Alkylrest der Formel CₙH₂ₙ₊₁ trägt, wobei n im Bereich von 1 bis 5 liegt; und
falls R'o eine Alkylkette ist, es sich bei R₀ um H oder um eine Alkylkette handelt, die 1 bis 11 eventuell verzweigte Kohlenstoffatome aufweist.

6. Verfahren nach Anspruch 5, wobei es sich, in der Formel II des Olefins, bei B' um H handelt und -B die Formel -(CR₂R₃)ₘ-CH₃ hat, wobei es sich bei den R₂ und den R₃, die identisch oder unterschiedlich sind, entweder um H oder um einen gesättigten Alkylrest handelt, der 1 bis 5 Kohlenstoffatome aufweist, wobei die R₂ einesteils und die R₃ andernteils, die an die Kohlenstoffatome der Kette gebunden sind, zueinander identisch oder unterschiedlich sind, und es sich bei mindestens einem von den R₂ und den R₃ um einen gesättigten Alkylrest handelt, wobei der Kohlenstoff, der diesen Rest trägt, sich in der Kette vorzugsweise an der Position ω-i befindet, wobei i > 1.

7. Verfahren nach einem der vorhergehenden Ansprüche, wobei die mindestens eine Verzweigung des Olefins sich vorzugsweise näher zur C=C-Doppelbindung als zum Ende der diese Verzweigung tragenden Kette befindet.

8. Verfahren nach einem der Ansprüche 5 bis 7, wobei jedes Kohlenstoffatom der Doppelbindung von Formel II: RoRo'C=CBB' eine einzige Alkylkette trägt.

9. Verfahren nach einem der vorhergehenden Ansprüche, wobei die Kohlenstoffatome der mindestens einen Doppelbindung des Olefins auf einer Seite der Doppelbindung an eine Kette aus höchstens zwei weiteren Kohlenstoffatomen gebunden sind, und vorzugsweise vom Typ Alken-1, Alken-2 oder Alken-3 sind.

10. Verfahren nach einem der vorhergehenden Ansprüche, wobei man mindestens einen Ruthenium-Carben-Katalysator verwendet, der aus geladenen oder ladungsfreien Katalysatoren der folgenden allgemeinen Formel gewählt ist:
(X₁)ₐ (X₂)_{b}Ru(Carben-C) (L₁)_{c}(L₂)_{d}(L₃)ₑ
wobei:
a, b, c, d und e Ganzzahlen sind, die identisch oder unterschiedlich sind, wobei a und b sind: 0, 1 oder 2; c, d und e sind: 0, 1, 2, 3 oder 4;
X₁ und X₂, die identisch oder unterschiedlich sind, jeweils einen geladenen oder ladungsfreien, mono- oder multi-chelatisierenden Liganden repräsentieren, wie beispielsweise Halogenide, Sulfat, Carbonat, Carboxylate, Alkoholate, Phenolate, Amide, Tosylat, Hexafluorphosphat, Tetrafluorborat, Bis-Triflylamid, ein Alkyl, Tetraphenylborat und Derivate davon, wobei X₁ oder X₂ an L₁ oder L₂ oder an Carben-C gebunden sein können, so dass ein zweizähniger Ligand oder Chelat am Ruthenium gebildet wird; und
L₁, L₂ und L₃, die identisch oder unterschiedlich sind, Elektronendonator-Liganden sind, wie beispielsweise Phosphin, Phosphit, Phosphonit, Phosphinit, Arsin, Stilbin, ein Olefin oder ein Aromat, eine Carbonylverbindung, ein Ether, ein Alkohol, ein Amin, ein Pyridin oder ein Derivat davon, ein Imin, ein Thioether, oder ein heterocyclisches Carben, wobei L₁, L₂ oder L₃ an das Carben-C gebunden sein können, so dass ein zweizähniger Ligand oder Chelat, oder ein dreizähniger Ligand gebildet wird.

11. Verfahren nach Anspruch 10, **gekennzeichnet durch** Verwendung eines Katalysators, der einen gesättigten NHC-Liganden aufweist, welcher durch Phenyle substituiert ist, die durch Mesityl (Mes)- oder Isopropyl (iPR)-Gruppierungen substituiert sind, vorzugsweise die Verwendung des Katalysators der Formel:

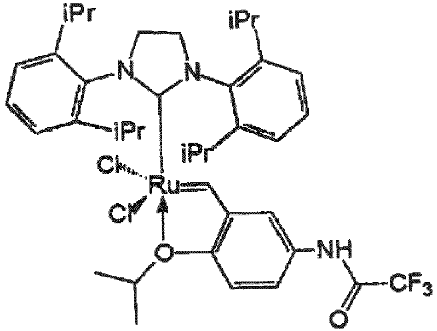

12. Verfahren nach einem der vorhergehenden Ansprüche, **dadurch gekennzeichnet, dass** es in einem flüssigen Milieu bei einer Temperatur im Bereich von 20 bis 160 °C und bei einem Druck im Bereich von 1 bis 30 bar durchgeführt wird.

13. Verfahren nach Anspruch 8, **dadurch gekennzeichnet, dass** es bei einer Temperatur im Bereich von 20 bis 120 °C und bei einem Druck im Bereich von 1 bis 10 bar durchgeführt wird.

14. Verfahren nach einem der vorhergehenden Ansprüche, **dadurch gekennzeichnet, dass** die Reaktion unter Vorliegen eines Lösungsmittels wie etwa Toluol, Xylol oder Dichlormethan durchgeführt wird.

15. Verfahren nach einem der vorhergehenden Ansprüche, **dadurch gekennzeichnet, dass** es weiter, nach dem Erzielen der Verbindung III, welche mindestens eine C=C-Doppelbindung trägt, mindestens eine der folgenden Reaktionen beinhaltet:
Hydrierung der mindestens einen C=C-Doppelbindung, die gemäß dem Typ der Hydrierung für die Ester zu gesättigten Estern, gesättigten Alkoholen, sogar verzweigten Paraffinen, und für Nitrile zu verzweigten Aminen führt;
Hydrolyse von Ester, die zu einer Säure führt;
Nitrilierung von Säure oder Ester, gemäß dem zwischenzeitlich eine Hydrolyse vorliegt oder nicht, und danach eine eventuelle Umwandlung des Nitril in ein Amin durch Hydrierung;
Epoxidierung der mindestens einen C=C-Doppelbindung, und zwar mit oder ohne Öffnung des Epoxidrings, um Diole, Carbonate, Ester zu bilden, was zum Epoxid einer verzweigten Kette führt; und/oder
erneuter Schritt einer Metathese, insbesondere in dem Fall, bei dem die Metathese mit verzweigten Olefin bei einem Diester vorgenommen wurde.

16. Verwendung einer fetten ungesättigten verzweigten Verbindung, die der Formel III A-CH=CB'-B entspricht und die durch das Verfahren nach einem der Ansprüche 1 bis 14 erzielt wurde, zur Herstellung mindestens eines der folgenden Produkte: dielektrische Fluide, Spezialtenside, Emulgatoren, Reibungsmittel, Antistatik-Additive, Antibeschlag-Additive, Entformungsmittel, Pigment-Dispergiermittel, Hochleistungsschmiermittel, Wachse und Wachs-Emulgatoren, Additive zur Umsetzung von Polymeren, PVC-Stabilisatoren, Tinten, Harze, Anstreichfarben, Lacke, Lösungsmittel, Lippenstift, Hautcreme, Deodorants, insbesondere in Stiftform, Haarfärbemittel, Shampoos und andere flüssige Seifen, Rasierschaum, Waschmittel, Reinigungsmittel, Textilweichspüler, und Gemische aus diesen.

## Claims

1. A process for synthesizing a branched unsaturated fatty compound
of formula III: A-CH=CB'-B
in which:
A is a radical consisting of an linear, optionally unsaturated, alkyl chain, comprising from 1 to 20 carbon atoms and comprising an acid, ester (of a monoalcohol or of a polyol) or nitrile end function,
B' is H or an alkyl chain comprising from 1 to 10 carbon atoms, and
B is:
- if B' is an alkyl chain, B is an alkyl, optionally branched, chain comprising from 1 to 11 carbon atoms,
- if B' is H, B is an alkyl chain comprising from 3 to 11 carbon atoms bearing at least
one branched alkyl radical of formula CₙH₂ₙ₊₁ with n in the range from 1 to 5,
said process comprising metathesis in the presence of a metathesis catalyst between:
- on the one hand, a linear unsaturated fatty compound of formula I:
R-(CH₂)a-[(CH=CH)-(CH₂)]_{b}-(CH₂)_{c}-R'
in which
R' is a radical -COOH, -COOR₁ or -CN, R₁ being a monoalcohol or a polyol residue, such as diol or triol,
R is H or an acid, monoalcohol ester or nitrile function, and
a, b, c are integers such that 0 ≤ a ≤ 11; 1 ≤ b ≤ 6; 2 ≤ c ≤ 12, the sum a + 3*b + c + 1 being in the range from 8 to 36, preferably from 8 to 24, preferably from 10 to 22 and even more preferably from 10 to 18; and
- on the other hand, a branched olefin II comprising from 4 to 23 carbon atoms, in which the main chain comprises at least one branching of formula CₙH₂ₙ₊₁ with n in the range from 1 to 5 when B' is H.

2. The process as claimed in claim 1, in which the linear unsaturated fatty compound I is chosen from: monounsaturated or polyunsaturated fatty acids or fatty acid esters, including mono-, di- and triglycerides, and nitrile derivatives, of the following fatty acids: oleic, petroselinic, linoleic, ricinoleic, gadoleic, gondoic, vaccenic, linolenic, palmitoleic, erucic, nervonic, and mixtures thereof.

3. The process as claimed in either of claims 1 and 2, in which compound I is chosen from:
- nitrile derivatives obtained by nitrilation of an ester or acid function of a fatty ester or fatty acid,
- difunctional molecules bearing acid, ester or nitrile functions at each end of the molecule,
- triglycerides optionally subjected to a chemical reaction, such as methanolysis, ethanolysis or metathesis with a C2-C4 light olefin, and also
- functional molecules comprising from 8 to 12 carbon atoms and preferably ω, ω-1 or ω-2 unsaturated, derived from cross metathesis of fatty acids/esters with a light olefin, such as ethylene, propylene or butene.

4. The process as claimed in any one of the preceding claims, in which the branched olefin II used for the metathesis reaction preferably contains at least 5 carbon atoms, and preferably comprises from 7 to 12, preferably from 8 to 12 carbon atoms in total.

5. The process as claimed in any one of the preceding claims, in which the branched olefin II is of formula: R₀R₀'C=CBB' in which:
B' is H or an alkyl chain comprising from 1 to 10 carbon atoms, and
- if B' is an alkyl chain, B is an alkyl, optionally branched, chain comprising from 1 to 11 carbon atoms,
- if B' is H, B is an alkyl chain comprising from 3 to 11 carbon atoms including at least one carbon atom bearing an alkyl radical of formula CₙH₂ₙ₊₁ with n in the range from 1 to 5, and
R'₀ is H or an alkyl chain comprising from 1 to 10 carbon atoms, and
- if R'₀ is H, R₀ is H, CH₃, C₂H₅ or an alkyl chain comprising from 3 to 11 carbon atoms including at least one carbon atom bearing an alkyl radical of formula CₙH₂ₙ₊₁ with n in the range from 1 to 5, and
- if R'₀ is an alkyl chain, R₀ is H or an alkyl, optionally branched, chain comprising from 1 to 11 carbon atoms.

6. The process as claimed in claim 5, in which, in formula II of the olefin, B' is H and -B is of formula -(CR₂R₃)ₘ-CH₃ in which R₂ and R₃, which may be identical or different, are either H or a saturated alkyl radical comprising from 1 to 5 carbon atoms, the radicals R₂, on the one hand, and the radicals R₃, on the other hand, linked to the carbon atoms of the chain being identical to or different from each other, and at least one of the radicals R₂ and R₃ being a saturated alkyl radical, the carbon bearing said radical preferably being in position ω-i in the chain, i being > 1.

7. The process as claimed in any one of the preceding claims, in which said at least one branching of the olefin is preferably located closer to the double bond C=C than the end of the chain which bears said branching.

8. The process as claimed in any one of claims 5 to 7, in which each carbon atom of the double bond of formula II: R₀R₀'C=CBB' bears only one alkyl chain.

9. The process as claimed in any one of the preceding claims, in which the carbon atoms of said at least one double bond of the olefin are linked on one side of the double bond to a chain of not more than 2 other carbon atoms, and are preferably of the 1-alkene, 2-alkene or 3-alkene type.

10. The process as claimed in any one of the preceding claims, in which use is made of at least one ruthenium-carbene catalyst chosen from the charged or uncharged catalysts of general formula:
(X₁)ₐ (X₂)_{b}Ru(carbene C) (L₁)_{c}(L₂)_{d} (L₃)ₑ
in which:
- a, b, c, d and e are integers, which may be identical or different, with a and b equal to 0, 1 or 2; c, d and e equal to 0, 1, 2, 3 or 4;
- X₁ and X₂, which may be identical or different, each represent a charged or uncharged and monochelating or polychelating ligand such as halides, sulfate, carbonate, carboxylates, alkoxides, phenolates, amides, tosylate, hexafluorophosphate, tetrafluoroborate, bis(triflyl)amide, an alkyl, tetraphenylborate and derivatives, X₁ or X₂ being possibly bonded to L₁ or L₂ or to the carbene C so as to form a bidentate or chelate ligand on the ruthenium; and
- L₁, L₂ and L₃, which may be identical or different, are electron-donating ligands, such as phosphine, phosphite, phosphonite, phosphinite, arsine, stilbene, an olefin or an aromatic compound, a carbonyl compound, an ether, an alcohol, an amine, a pyridine or derivative, an imine, a thioether, or a heterocyclic carbine, L₁, L₂ or L₃ being possibly bonded to the carbene C so as to form a bidentate or chelate ligand, or a tridentate ligand.

11. The process as claimed in claim 10, **characterized by** the use of a catalyst comprising a saturated NHC ligand substituted with phenyls substituted with mesityl (Mes) or isopropyl (iPr) groups, preferably the use of the catalyst of formula:

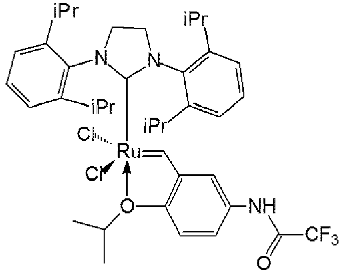

12. The process as claimed in any one of the preceding claims, **characterized in that** it is performed in liquid medium at a temperature in the range from 20 to 160°C and at a pressure in the range from 1 to 30 bar.

13. The process as claimed in claim 8, **characterized in that** it is performed at a temperature in the range from 20 to 120°C and at a pressure in the range from 1 to 10 bar.

14. The process as claimed in any one of the preceding claims, **characterized in that** the reaction is performed in the presence of a solvent such as toluene, xylenes or dichloromethane.

15. The process as claimed in any one of the preceding claims, **characterized in that** it also comprises, after the production of compound III bearing at least one double bond C=C, at least one of the following reactions:
• hydrogenation of said at least one double bond C=C, which leads, depending on the type of hydrogenation, for the esters, to saturated esters, saturated alcohols, or even branched paraffins, and, for the nitriles, to branched amines;
• hydrolysis of ester, which leads to an acid;
• nitrilation of acid or ester, depending on whether or not there is an intermediate hydrolysis, followed by optional conversion of the nitrile into amine by hydrogenation;
• epoxidation of said at least one double bond C=C, with or without opening of the epoxy ring, to form diols, carbonates or esters, leading to the epoxy of a branched chain; and/or
• new step of metathesis in particular in the case where the metathesis with the branched olefin was performed on a diester.

16. The use of the branched unsaturated fatty compound of formula III A-CH=CB'-B obtained by the process as claimed in any one of claims 1 to 14, for manufacturing at least one of the following products: dielectric fluids, specialty surfactants, emulsifiers, friction agents, antistatic additives, antifogging additives, mold-release agents, pigment dispersants, high-performance lubricants, waxes and wax emulsifiers, polymer transformation additives, PVC stabilizers, inks, resins, paints, varnishes, solvents, lipsticks, skin creams, deodorants, especially in stick form, hair dyes, shampoos and other liquid soaps, shaving foams, detergents, cleaning agents, textile softeners, and mixtures thereof.
